Europäisches Patentamt

European Patent Office

Office européen des brevets

(11) Veröffentlichungsnummer: **0 002 201**
B1

# (12) EUROPÄISCHE PATENTSCHRIFT

(45) Veröffentlichungstag der Patentschrift:
27.01.82

(21) Anmeldenummer: 78101350.3

(22) Anmeldetag: 11.11.78

(51) Int. Cl.³: **A 61 K 6/08**, A 61 C 13/08, A 61 C 13/22, C 08 L 33/10

(54) Dentalformkörper auf Basis von Polymethacrylaten, Verfahren zu ihrer Herstellung sowie Verwendung von Polymethacrylatmassen.

(30) Priorität: 25.11.77 DE 2752611

(43) Veröffentlichungstag der Anmeldung:
13.06.79 Patentblatt 79/12

(45) Bekanntmachung des Hinweises auf die Patenterteilung:
27.01.82 Patentblatt 82/4

(84) Benannte Vertragsstaaten:
BE CH DE FR GB NL SE

(56) Entgegenhaltungen:
DE-A-2 003 365
DE-B-1 769 840
GB-A-1 324 432

(73) Patentinhaber: BAYER AG, Zentralbereich Patente, Marken und Lizenzen, D-5090 Leverkusen 1, Bayerwerk (DE)

(72) Erfinder: Süllng, Carlhans, Dr., Carl-Leverkus-Strasse 10, D-5068 Odenthal (DE)
Erfinder: Bailé, Gerhard, Dr., Nietzsche-Strasse 14, D-5090 Leverkusen (DE)
Erfinder: Leusner, Bernd, Dr., Grüner Weg 148, D-5090 Leverkusen (DE)
Erfinder: Schulz, Hans-Hermann, Dr., Am Neulandkreuz 23, D-5653 Leichlingen (DE)
Erfinder: Walkowiak, Michael, Dr., Albertus-Magnus-Strasse 10, D-5090 Leverkusen 1 (DE)

### Dentalformkörper auf Basis von Polymethacrylaten, Verfahren zu ihrer Herstellung sowie Verwendung von Polymethacrylatmassen

Gegenstand der Erfindung sind Dentalformkörper, wie Prothesen, Kronen oder Brücken, mit verbesserten mechanischen Eigenschaften.

Zahnprothesen aus Kunststoff werden in den meisten Fällen nach dem Pulver-Flüssigkeitsverfahren hergestellt (DE-PS 737 058).

Bei dieser Arbeitsweise wird ein Perlpolymerisat auf der Basis von Polymethacrylaten mit Methacrylaten, wie z. B. Methylmethacrylat, zu einem Teig verarbeitet, indem man 2 bis 3 Teile Pulver mit 1 Teil Flüssigkeit anrührt. Das Monomer ist vor der Teigherstellung mit einem Peroxid versetzt worden, so daß der Teig nach Eingeben in eine Hohlform durch Erhitzen unter Polymerisation des Monomeren ausgehärtet werden kann.

Die leichte Durchführbarkeit des Herstellungsverfahrens für Zahnprothesen, Kronen und Brücken bewirkt, daß das Pulver-Flüssigkeitsverfahren zur Standardtechnik der Herstellung von Kunststoffzahnprothesen geworden ist. Es ist weiterhin bekannt, die Verarbeitbarkeit von Dentalperlen beim Pulver-Flüssigkeitsverfahren dadurch zu verbessern, daß man Polymethylacrylatpulver oder bevorzugt Polymethylmethacrylatperlen einer definierten Korngröße verwendet, und es ist außerdem bekannt, die Verarbeitungsweise von Dentalperlen dadurch zu verbessern, daß man nicht Polymethylmethacrylatperlen, sondern Perlen aus Copolymerisaten des Methylmethacrylates mit überwiegendem Anteil an copolymerisierten Methacrylsäuremethylester als Pulver verwendet. Durch diese Variationen gelingt es, die gewünschte rasche Verarbeitbarkeit bei der ebenfalls gewünschten großen Verarbeitungsbreite einzustellen.

Nachteilig für die aus den nach dem Pulver-Flüssigkeitsverfahren auf Basis von Polymethylmethacrylaten hergestellten Zahnprothesen, Kronen und Brücken ist es, daß die mechanischen Werte des Rohstoffes für viele Konstruktionen nicht befriedigen. Besonders die Zähigkeiten der Kunststoffe reichen bei Belastungen in vielen Fällen für Prothesen, Kronen und Brücken nicht aus. Eine Verbesserung der Schlagzähigkeit des Kunststoffes würde bewirken, daß die Bruchanfälligkeit der Prothesen geringer wird und daß auch der Reinigungsvorgang deshalb sicherer durchgeführt werden kann.

Es wurde gefunden, daß nach dem Pulver-Flüssigkeitsverfahren hergestellte Dentalformkörper, wie Prothesen, Brücken und Kronen auf der Basis von Polymethacrylaten dann verbesserte mechanische Eigenschaften besitzen, wenn man als Pulver Polymethylmethacrylate verwendet oder mitverwendet, die durch 0,5 − 12 Gew.-% unvernetzte diaminverlängerte Polyurethane elastifiziert worden sind.

Das gleiche gilt auch für in dieser Weise hergestellte künstliche Zähne. Auch als Komponente von Reparaturmaterialien für Zahnprothesen, Brücken und Kronen sind die durch unvernetzte diaminverlängerte Polyurethane elastifizierten Polymethylmethacrylate geeignet.

Es ist bekannt, Polymethylmethacrylate dadurch zu elastifizieren, daß man die Polymerisation des Methylmethacrylates nach dem Verfahren einer Massepolymerisation bei gleichzeitiger Formgebung durchführt. Es war jedoch nicht zu erwarten, daß man Prothesen mit verbesserten Eigenschaften erhalten kann, wenn man nach dem Pulver-Flüssigkeitsverfahren arbeitet und als Pulver ein Polymethylmethacrylat verwendet, das als elastifizierende Komponente ein diaminverlängertes Polyurethan enthält.

Wie allgemein bekannt ist, sind Dentalkunststoffe, die nach dem Pulver-Flüssigkeitsverfahren erhalten werden, durch einen besonderen Aufbau gekennzeichnet. Im ausgehärteten Kunststoff liegt, durch spezielle Methoden nachweisbar, ein mehrphasiges System vor: Die ursprüngliche »Flüssigkeit« ist beim Anquellvorgang nur zum Teil in die Pulverpartikel eingedrungen. Ein großer — wenn nicht überwiegender — Anteil der Flüssigkeit polymerisiert als Phase für sich und füllt die Zwischenräume zwischen den gequollenen ursprünglichen Pulverpartikeln aus. Formkörper aus Polymethacrylaten oder modifizierten Polymethylmethacrylaten, die nach dem Pulver-Flüssigkeitsverfahren erhalten worden sind, unterscheiden sich im Aufbau damit wesentlich von Formkörpern aus Polymethylmethacrylaten, die über übliche Formgebungsverfahren erhalten wurden.

Aus DE-PS 940 493 ist es zwar bekannt, auch die mechanischen Werte von Formkörpern aus Methylmethacrylaten zu verbessern, indem man Mischungen verschiedener Polymerisate oder Copolymerisate als Pulverkomponenten verwendet. Zur Verbesserung der Dauerbiegefestigkeit wurden zum Beispiel Mischpolymerisate aus 80% Methylmethacrylat und 20% Butadien verwendet. Derartige Copolymerisate haben aber aufgrund des Butadiengehaltes eine schlechte Lichtechtheit.

Weiterhin ist es aus DE-PS 940 493 bekannt, nachchloriertes Polyvinylchlorid als Zusatz zu verwenden, um die Schlagbiegefestigkeit und die Dauerbiegefestigkeit von Formkörpern auf Basis von Methylmethacrylat-Polymerisaten, die nach dem Pulver-Flüssigkeitsverfahren erhalten worden sind, zu verbessern. Nachchlorierte Polyvinylchloride als Zusatz bewirken jedoch eine Abnahme der Verfärbungsbeständigkeit. Außerdem ist bei Verwendung aktiver Peroxyde oder höherer Polymerisationstemperaturen die Stabilität von nachchlorierten Polyvinylchloriden nicht ausreichend.

Formkörper für Dentalzwecke, wie Zahnprothesen, Brücken oder Kronen, auf Basis von organischen Kunststoffen, können nach verschiedenen Verfahrensweisen hergestellt werden.

So kann zum Beispiel der Kunststoff über ein Spritz- oder Extrusionsverfahren in den gewünschten Formkörper umgewandelt werden.

Die erfindungsgemäßen Zahnprothesen, Brükken, Kronen oder Zähne werden nach diesem Verfahren erhalten, indem man Polymethacrylate, die durch unvernetzte diaminverlängerte Polyurethane elastifiziert sind, gegebenenfalls im Gemisch mit üblichen »spritzfähigen« Polymethylmethacrylaten, über eine Spritz- oder über eine Extrusionsvorrichtung verformt.

Besonders vielseitig zur Herstellung von Zahnprothesen, Kronen oder Brücken ist jedoch das Pulver-Flüssigkeitsverfahren. Die erfindungsgemäßen Formkörper werden nach diesem Verfahren erhalten, indem ein Polymethacrylat, das durch unvernetztes diaminverlängertes Polyurethan elastifiziert ist, als Pulver verwendet wird. Diese Pulver können dadurch erhalten werden, daß Polymethacrylate, die durch unvernetzte diaminverlängerte Polyurethane elastifiziert sind, über einen Zerkleinerungsprozeß in ein sogenanntes »Splitteracrylat« umgewandelt werden. Besonders gute Ergebnisse werden jedoch erhalten, wenn solche elastifizierte Polymethacrylatpulver verwendet werden, die nach der Verfahrensweise einer Perlpolymerisation hergestellt worden sind, und die aus

(A) 88 bis 99,5 Gew.-% eines Polymerisats aus polymerisierten Einheiten von mindestens einem Methacrylsäureester mit 1 bis 10 C-Atomen in der aliphatischen, gesättigten Alkolkomponente, wobei im Methacrylester-Polymerisat bis zu 30 Gew.-% copolymerisierte Einheiten mindestens eines Monomeren aus der Gruppe Acrylsäureester mit 1 bis 10 C-Atomen in der aliphatischen, gesättigten Alkolkomponente, Hydroxyalkylester der Acrylsäure oder Methacrylsäure mit 2 bis 4 C-Atomen in der Alkylgruppe, Styrol, Vinylacetat, (Meth)Acrylamid, (Meth)Acrylsäure und/oder Itaconsäure enthalten sein können, und
(B) 0,5 bis 12 Gew.-% eines unvernetzten diaminverlängerten Polyurethans, welches aus

   1) mindestens einer Dihydroxyverbindung,
   2) Diisocyanaten aus der Gruppe der

     a) aliphatischen Diisocyanate mit einem verzweigten Kohlenstoffgerüst von 7 bis 36 C-Atomen,
     b) cycloaliphatischen Diisocyanate und
     c) durch radikalische Pfropfcopolymerisation mit Vinylmonomeren modifizierten aliphatischen oder cycloaliphatischen Diisocyanate

   3) aliphatischen oder cycloaliphatischen Diaminen und monofunktionellen, gegebenenfalls ungesättigte Gruppen aufweisenden Kettenabbrechern erhalten worden ist,

bestehen.

Die erfindungsgemäße Verwendung der elastifizierten Polymerisatperlen bringt neben der besseren Verarbeitbarkeit gegenüber den Splitteracrylaten zusätzlich den Vorteil, daß die elastifizierende Komponente besser gegenüber einem Abbau durch und generell gegen eine Einwirkung von Komponenten des Mundmilieus abgeschirmt ist. Bei den Dentalperlen wird das als getrennte Phase vorhandene unvernetzte diaminverlängerte Polyurethan von der Grundsubstanz der Dentalperlen, dem Polymethacrylat, umhüllt und so vor einer Einwirkung abgeschirmt. Außerdem werden auch die Dentalperlen selbst wieder in eine Matrix von Polymethacrylat eingebettet und damit abgeschirmt.

Aus DE-OS 2 003 365 ist zwar bereits die Verbesserung der mechanischen Eigenschaften von diaminverlängerte Polyurethane enthaltenden Polymethacrylaten bekannt. Zur Erreichung dieses Zieles ist ein vernetztes Polyurethan erforderlich, das in einer definierten Teilchengrößenverteilung vorliegen muß.

Dagegen werden erfindungsgemäß lineare, lösliche, d. h. unvernetzte Polyurethane verwendet.

Es war nicht vorherzusehen, daß durch die Verwendung dieser Polyurethane eine viel größere Verbesserung der Elastifizierung erreicht wird als nach dem Verfahren der genannten Publikation.

Eine besondere Ausführungsform bei der erfindungsgemäßen Verfahrensweise zur Herstellung von Prothesen, Kronen oder Brücken nach dem Pulver-/Flüssigkeitsverfahren besteht darin, die gewünschte Verarbeitbarkeit und die erforderliche Verarbeitungsbreite dadurch einzustellen, daß man die elastifizierten Dentalperlen in einer definierten Korngröße verwendet, oder daß man das Anquellverhalten der Polymerisatperlen durch Verwendung von Comonomeren bei der Perlpolymerisation einstellt. Ganz besonders vorteilhaft ist es jedoch, die Kenngrößen Verarbeitbarkeit und Verarbeitungsbreite, die für eine zahntechnische Handhabung besonders wichtig sind, durch Zusatz von nicht elastifizierten Perlen einzustellen. Es war überraschend, daß die gute elastifizierende Wirksamkeit der Dentalperlen nicht gemindert wird, wenn letztere im Gemisch mit üblichen Dentalperlen verwendet werden. Die technisch günstigsten Mischungsverhältnisse müssen allerdings von Fall zu Fall ermittelt werden und hängen von der Konstruktion und von der Funktion der Prothese oder Brücke ab.

Unter Polymethacrylaten im Sinne der vorliegenden Erfindung werden Polymerisationsprodukte von Methacrylsäureestern verstanden. In den meisten Fällen ist Methacrylsäuremethylester die Hauptkomponente, jedoch werden brauchbare Ergebnisse auch mit polyfunktionellen Estern der Methacrylsäure erhalten, und für spezielle Zwecke geben zum Beispiel Bis-GMA (Bis-4-(2-hydroxy-3-methacryloyloxypropoxy)-phenyl-dimethylmethan) oder dessen Abwandlungsprodukte und auch die in der US-PS 3 730 947 genannten Comonomeren gute Ergebnisse.

Diaminverlängerte unvernetzte Polyurethane gemäß der vorliegenden Erfindung sind Polyurethan-Polyharnstoffelastomere, die als Hartsegmente Harnstoffgruppen in einer Polyurethankette enthalten. Die Harnstoffgruppen werden durch Verwendung von Diaminen als Kettenverlängerer in die Polyurethanketten eingeführt.

Die Herstellung der verwendeten Polyurethanharnstoffelastomeren erfolgt nach in der Polyurethanchemie üblichen Methoden durch Polyaddition nach dem sogenannten one shot-Verfahren, bei dem alle Komponenten zusammengegeben und in einem Schritt zum fertigen Polyurethanharnstoff umgesetzt werden, oder nach dem Zwei-Stufen- oder Präpolymer-Verfahren. Bei letzterem, hier bevorzugtem Verfahren erzeugt man aus der oder den Diolkomponenten und einem stöchiometrischen Überschuß des Diisocyanats zunächst ein Vorpolymerisat mit endständigen Isocyanatgruppen, das man in zweiter Stufe durch Umsetzung mit dem Diamin verlängert.

Vertreter der zu verwendenden Ausgangskomponenten für die Herstellung der erfindungsgemäß zu verwendenden Polyurethane sind z. B. in High Polymers, Vol. XVI, »Polyurethane, Chemistry and Technology«, verfaßt von Saunders—Frisch, Interscience Publishers, New York, London, Band I, 1962, Seiten 32—42 und Seiten 44—54, und Band II, 1964, Seiten 5 und 6 und 198 und 199, sowie im Kunststoff-Handbuch, Band VII, Vieweg—Höchtlen, Carl-Hanser-Verlag, München, 1966, z. B. auf den Seiten 45 bis 71, beschrieben.

Die Wahl des geeigneten Diisocyanates wird durch die Forderung nach hoher Lichtechtheit, welche durch Verwendung aliphatischer oder alicyclischer Diisocyanate erreicht wird sowie weiterhin durch die Tatsache eingeschränkt, daß die als Lösungsmittel für das Polyurethanharnstoffelastomer verwendeten Acryl- und Methacrylsäureester, insbesondere das Methylmethacrylat, thermodynamisch schlechte Lösungsmittel für das Elastomere darstellen. Dies äußert sich einmal in der steilen Viskositätszunahme mit steigendem Gehalt an Harnstoffgruppen in der Polymerkette, zum anderen führt die Verwendung von aliphatischen Diisocyanaten mit linearer Kettenstruktur zu getrübten Lösungen. Dies wird darauf zurückgeführt, daß die ausgebildeten Polyharnstoffsegmente wegen der fehlenden sterischen Hinderung so stark assoziieren, daß sie gewissermaßen aus der Lösung auskristallisieren. Dadurch fallen auch die aus den Lösungen resultierenden Polymerisate getrübt an. Dies ist zum Beispiel bei dem technisch am meisten verwandten aliphatischen Diisocyanat, dem Hexamethylendiisocyanat, der Fall. Deshalb kommen für die Herstellung der erfindungsgemäßen Polyurethanharnstoffelastomerlösungen solche aliphatischen Diisocyanate in Betracht, die eine komplexere, nichtlineare Struktur besitzen und so einen Kompromiß zwischen der an sich erwünschten Assoziation der Harnstoffsegmente untereinander und der optischen Beschaffenheit des Produktes zu finden helfen. Dies sind:

A) aliphatische Diisocyanate mit einem verzweigten Kohlenstoffgerüst von 7 bis 36 C-Atomen, z. B. 2.2.4- oder 2.4.4-Trimethylhexan-1.6-diisocyanat oder technische Gemische daraus, von Estern des Lysins abgeleitete Diisocyanate, oder Diisocyanate auf der Basis dimerisierter Fettsäuren, die in bekannter Weise durch Überführung derartiger Dicarbonsäuren mit bis zu 36 C-Atomen in die entsprechenden Diamine und anschließende Phosgenierung dargestellt werden,

B) cycloaliphatische Diisocyanate, z. B. 1,3-Cyclobutandiisocyanat, 1.3- und 1.4-Cyclohexandiisocyanat, 2.4- oder 2.6-Diisocyanato-1-methylcyclohexan oder 4.4'-Diisocyanatodicyclohexylmethan, entweder in Form der reinen geometrischen Isomeren oder technischer Gemische derselben, ferner das 1-Isocyanato-3.3.5-trimethyl-5-isocyanatomethylcyclohexan (Isophorondiisocyanat) sowie schließlich

C) durch radikalische Pfropfcopolymerisation mit Vinylmonomeren modifizierte aliphatische oder cycloaliphatische Diisocyanate, die in der Weise erhalten werden, daß man in Gegenwart von 100 Teilen des Diisocyanates von 10 bis zu 100 Teilen, vorzugsweise von 20 bis zu 75 Teilen, eines Vinylmonomeren, vorzugsweise Methylmethacrylat, mit Hilfe eines radikalischen Polymerisationsinitiators, z. B. eines organischen Peroxids, wie Benzoylperoxid, tert.-Butyleroctoat usw. oder einer aliphatischen Azoverbindung wie Azoisobutyronitril zur Polymerisation bringt. Als Pfropfsubstrat eignen sich neben den bereits genannten Diisocyanaten auch aliphatische Diisocyanate mit linearer Kohlenstoffkette, zum Beispiel das Hexamethylendiisocyanat. Es hat sich gezeigt, daß in dieser Weise modifizierte aliphatische Diisocyanate zu Polyurethenharnstoffelastomeren führen, die in monomerem Methylmethacrylat klar löslich sind, und bei richtiger Angleichung der Brechungsindizes von Polymer- und Zähphase klare Polymerisate ergeben.

Vorzugsweise verwendet werden das Isophorondiisocyanat und durch Pfropfcopolymerisation mit Methylmethacrylat modifiziertes Hexamethylendiisocyanat oder Isophorondiisocyanat mit einem Polymerisatgehalt bis zu 50%, vorzugsweise bis zu 40%.

Geeignete längerkettige Diole mit 2 endständigen Hydroxylgruppen im Molekül sind vorzugsweise Polyester, Polyäther, Polyacetale, Polycarbonate, Polyesteramide und Polyamide vom Molekulargewicht 400–6000, die vorzugsweise eine Glasübergangstemperatur $\leq -20°C$ aufweisen. Polyester- und Polyätherdiole sind besonders bevorzugt.

Die in Frage kommenden Hydroxylgruppen aufweisenden Polyester sind zum Beispiel Umsetzungsprodukte von zweiwertigen Alkoholen mit zweiwertigen Carbonsäuren. Anstelle der freien Polycarbonsäuren können auch die entsprechenden Polycarbonsäureanhydride oder entsprechende Polycarbonsäureester von niedrigen Alkoholen oder deren Gemische zur Herstellung der Polyester verwendet werden. Die Polycarbonsäuren können aliphatischer, cycloaliphatischer, aromatischer und/oder heterocyclischer Natur sein und gegebenenfalls, z. B. durch Halogenatome, substituiert und/oder ungesättigt sein. Als Beispiele hierfür seien genannt: Bernsteinsäure, Adipinsäure, Korksäure, Azelainsäure, Sebacinsäure, Phthalsäure, Isophthalsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, dimere Fettsäuren wie Ölsäure, Terephthalsäuredimethylester, Terephthalsäure-bis-glykolester. Als mehrwertige Alkohole kommen z. B. Äthylenglykol, Propylenglykol-(1,2) und -(1,3), Butylenglykol-(1,4)- und -(2,3), Hexandiol-(1,6), Octandiol-(1,8), Neopentylglykol, Cyclohexandimethanol (1,4-Bis-hydroxymethylcyclohexan), 2-Methyl-1,3-propandiol, ferner Diäthylenglykol, Triäthylenglykol, Tetraäthylenglykol, Polyäthylenglykole, Dipropylenglykol, Polypropylenglykole, Dibutylenglykol und Polybutylenglykole in Frage. Auch Polyester aus Lactonen, z. B. $\varepsilon$-Caprolacton oder Hydroxycarbonsäuren, z. B. $\omega$-Hydroxycapronsäure, sind einsetzbar.

Auch die in Frage kommenden zwei Hydroxylgruppen aufweisenden Polyäther sind solche der an sich bekannten Art und werden z. B. durch Polymerisation von Epoxiden wie Äthylenoxid, Propylenoxid, Butylenoxid, Tetrahydrofuran, Styroloxid oder Epichlorhydrin mit sich selbst, z. B. in Gegenwart von $BF_3$, oder durch Anlagerung dieser Epoxide, gegebenenfalls im Gemisch oder nacheinander, an Startkomponenten mit reaktionsfähigen Wasserstoffatomen wie Alkohole oder Amine, z. B. Wasser, Äthylenglykol, Propylenglykol-(1,3) oder -(1,2), 4,4'-Dihydroxy-diphenylpropan oder Anilin hergestellt. Besonders sind solche Polyäther bevorzugt, die überwiegend (bis zu 90 Gew.-%, bezogen auf alle vorhandenen OH-Gruppen im Polyäther) primäre OH-Gruppen aufweisen.

Als Polyacetale kommen z. B. die aus Glykolen, wie Diäthylenglykol, Triäthylenglykol, 4,4'-Dioxäthoxy-diphenyldimethylmethan, Hexandiol und Formaldehyd herstellbaren Verbindungen in Frage. Auch durch Polymerisation cyclischer Acetale lassen sich erfindungsgemäß geeignete Polyacetale herstellen.

Als Hydroxylgruppen aufweisende Polycarbonate kommen solche der an sich bekannten Art in Betracht, die z. B. durch Umsetzung von Diolen wie Propandiol-(1,3), Butandiol-(1,4) und/oder Hexandiol-(1,6), Diäthylenglykol, Triäthylenglykol, Tetraäthylenglykol mit Diarylcarbonaten, z. B. Diphenylcarbonat oder Phosgen, hergestellt werden können.

Geeignete Diamine zur Kettenverlängerung sind Alkylendiamine mit 2 bis 36 C-Atomen, z. B. Hexamethylendiamin, Undecamethylendiamin, 2.2.4- oder 2.4.4-Trimethyl-1.6-diaminohexan oder technische Gemische daraus, von dimeren Fettsäuren mit bis zu 36 C-Atomen abgeleitete Diamine, ferner cycloaliphatische Diamine mit 5 bis 25 C-Atomen, zum Beispiel die verschiedenen Diaminocyclohexane, Diaminohexahydro-toluole und Diaminodicyclohexylmethane, entweder in Form der reinen Stellungs- und geometrischen Isomeren oder als technische Isomerengemische sowie das 1-Amino-3,3,5-trimethyl-5-aminomethylcyclohexan (Isophorondiamin). Letzteres Diamin wird bevorzugt verwendet.

Als Kettenabbrecher für die nach der Kettenverlängerung etwa noch vorhandenen freien Isocyanatgruppen dienen gegenüber Isocyanat monofunktionelle Verbindungen, zum Beispiel Alkohole oder Amine. Diese können gesättigt, was oft bevorzugt ist, oder olefinisch ungesättigt sein. Im letzteren Fall können die so in den Polyurethanharnstoff eingeführten ungesättigten Gruppen an der Polymerisation des Methylmethacrylates teilnehmen, wodurch eine Verknüpfung zwischen Polymer- und Zähphase erreicht wird. Geeignete und bevorzugte gesättigte Kettenabbrecher sind zum Beispiel die niederen aliphatischen Alkohole, vorzugsweise mit 1–4 C-Atomen, wie Methanol, Äthanol oder Butanol oder aliphatische Monoamine, vorzugsweise mit 1–6 C-Atomen, wie Butylamin, Dibutylamin oder Cyclohexylamin. Zur Einführung polymerisationsfähiger Doppelbindungen eignen sich zum Beispiel Allylalkohol (bevorzugt), Allylamin oder bevorzugt Hydroxyalkylester (mit 2–4 C-Atomen in der Alkylgruppe) $\alpha,\beta$-ungesättigter Carbonsäuren mit 3–5 C-Atomen, wie 2-Hydroxyäthylmethacrylat (bevorzugt).

Die Polyurethan-elastifizierten Polymethacrylate werden zu ihrer zahntechnischen Verarbeitung nach dem Pulver-/Flüssigkeitsverfahren mit einem Monomer zu einem Teig vermischt. Als Monomer dient vorzugsweise Methylmethacrylat. Zur Erhöhung der Lösungsmittelbeständigkeit und Abriebfestigkeit gibt man Monomere zu, die zwei oder mehr Doppelbindungen im Molekül enthalten, und die damit zu einer Vernetzung führen. Als Vernetzer kann man zum Beispiel die folgenden Verbindungen in Mengen von 0,1 bis 30 Gew.-%, vorzugsweise 1 bis 15 Gew.-%, zusetzen:

Äthylenglykoldimethacrylat, Triäthylenglykoldimethacrylat, Butandioldimethacrylat, Trimethylolpropantrimethacrylat, Bis-GMA, Methylenbisacrylamid, Triacrylformal sowie die in US-PS 3 730 947 genannten bifunktionellen Comonomere.

Die Härtung der erhaltenen Massen aus Perlpolymerisat und Monomer kann durch Radikale liefernde Startersysteme auf Basis von Peroxiden oder aliphatischen Azoverbindungen bewirkt werden. Geeignete Polymerisationsstarter sind zum Beispiel Diacylperoxide, wie zum Beispiel Dibenzoylperoxid, Alkylacylperoxide, wie zum Beispiel Tertiärbutylperpivalat, gegebenenfalls in Gegenwart von Beschleunigern, wie aromatischen tertiären Aminen, zum Beispiel alkylierten Anilinen, Toluidinen, Xylidinen. Als Beschleuniger können ferner Cobalt- oder Kupfersalze sowie Verbindungen aus der Gruppe der Barbiturate sowie Sulfinsäuren und Sulfone Verwendung finden.

Während die Härtung bei erhöhter Temperatur durch Peroxide, wie Dibenzoylperoxid, Chlorbenzoylperoxid, Toluylperoxid, oder Laurylperoxid, alleine oder durch Radikalstarter, wie beispielsweise Azisobuttersäurenitril oder Azoisobuttersäureester alleine durchgeführt werden kann, macht eine Härtung bei niedrigen Temperaturen den Zusatz von Beschleunigern erforderlich. Bei der Härtung bei erhöhter Temperatur benötigt man von 0,01 bis 2 Gew.-% an Polymerisationsstarter. Bei der Härtung bei niedrigen Temperaturen benötigt man 0,02 bis 5 Gew.-% an Polymerisationsstartern sowie 0,02 bis 5 Gew.-% an Beschleunigern.

### Beispiel 1

Dentalperlen werden durch ein Verfahren zur Perlpolymerisation von Methylmethacrylat in Gegenwart eines Polyurethans hergestellt.

Als Dispergator bei der Perlpolymerisation wurde ein Polyvinylpyrrolidon vom K-Wert 90 verwendet, als peroxydischer Starter ein Gemisch aus Lauroylperoxyd und Dicyclohexylpercarbonat im Verhältnis 1 : 1 in einer Menge von 0,73%, bezogen auf verwendetes Methylmethacrylat (Gew.-%). Das Methylmethacrylat enthielt 9,9% Polyurethan gelöst.

Bei dem Polyurethan handelt es sich um ein diaminverlängertes Polyesterpolyurethan auf der Basis von einem Gemisch aus zwei Polyesterdiolen A und B.

Polyesterdiol A besteht aus einem Polyester auf Basis von Adipinsäure, 1,6-Hexandiol und Neopentylglykol mit einer Hydroxylzahl 66.

Polyester B ist ein Polyester auf Basis von Äthylenglykol, Adipinsäure und Phthalsäureanhydrid mit einer Hydroxylzahl von 64.

Polyester A (0,35 Äquivalente) und Polyester B (0,15 Äquivalente) werden mit Isophorondiisocyanat (0,75 Äquivalente) umgesetzt, anschließend Verlängerung mit Isophorondiamin auf einen Verlängerungsgrad von 90% und Abbruch mit 2-Hydroxyäthylmethacrylat.

15 Gew.-Teile der in dieser Weise hergestellten Dentalperlen werden mit 0,25 Gew.-% Dibenzoylperoxid versetzt und mit 5,36 Gew.-Teilen einer Flüssigkeit aus 94 Gew.-% Methylmethacrylat und 6 Gew.-% Äthylenglykoldimethacrylat angeteigt. Aus diesem Teig werden 2 mm starke Platten gepreßt und anschließend polymerisiert.

Die Polymerisation wird folgendermaßen durchgeführt: Innerhalb von 30 Minuten wird das Wasserbad auf 70° C aufgeheizt, 30 Minuten wird die Temperatur konstant gehalten, dann auf 100° C aufgeheizt und diese Temperatur für weitere 30 Minuten konstant gehalten. Die Abkühlung der Küvette erfolgt im Wasserbad.

Nach dem Ausbetten werden aus der Platte die Prüfkörper ohne Aufheizung der Platte geschnitten. Die so erhaltenen Prüfkörper werden nach DIN 53 452 der Dynstatprüfung unterzogen.

Prüfergebnisse (jeweils Mittelwert aus 5 Prüfkörpern):

| | |
|---|---|
| Schlagzähigkeit | 507,7 N/cm² |
| Biegewinkel | 19° |
| Biegefestigkeit | 115,5 N/cm² |
| Kugeldruckhärte | |
| 10″ | 132,7 N/cm² |
| 60″ | 122,5 N/cm² |

### Vergleich

Als Kontrollversuch werden übliche Methylmethacrylatperlen mit 0,25 Gew.-% Dibenzoylperoxid mit einer Flüssigkeit, bestehend aus 94% Methylmethacrylat und 6 Gew.-% Äthylenglykoldimethacrylat, polymerisiert und der Festigkeitsprüfung nach DIN 53 452 unterzogen.

| | |
|---|---|
| Schlagzähigkeit | 194 N/cm² |
| Biegewinkel | 18° |
| Biegefestigkeit | 105,9 N/cm² |
| Kugeldruckhärte | |
| 10″ | 124,9 N/cm² |
| 60″ | 115,8 N/cm² |

### Patentansprüche

1. Dentalformkörper auf Basis von Polymethacrylaten, dadurch gekennzeichnet, daß man Polymethacrylate verwendet, die durch 0,5 bis 12 Gew.-% unvernetzte diaminverlängerte Polyurethane elastifiziert sind.

2. Verfahren zur Herstellung von Dentalformkörpern nach dem Pulver-/Flüssigkeitsverfahren, dadurch gekennzeichnet, daß man als Pulver feinteilige Polymethylmethacrylate verwendet, die durch 0,5 bis 12 Gew.-% unvernetzte diaminverlängerte Polyurethane elastifiziert sind.

3. Verfahren nach Anspruch 2, dadurch gekennzeichnet, daß als Pulver elastifizierte feinteilige Polymethacrylate verwendet werden, die in Form von Polymerisatperlen nach der Verfahrensweise einer Perlpolymerisation erhalten werden.

4. Verfahren nach Anspruch 2, dadurch gekennzeichnet, daß als Pulver elastifizierte Polymethylmethacrylate verwendet werden, die über ein Mahlverfahren als Splitteracrylate erhalten werden.

5. Verfahren nach Ansprüchen 3 und 4, dadurch gekennzeichnet, daß den elastifizierten Polymethylmethacrylaten zur Einstellung der gewünschten Verarbeitbarkeit und Verarbeitungsbreite nicht elastifizierte Methacrylsäuremethylester-Polymerisate zugemischt werden.

6. Verwendung von durch 0,5 bis 12 Gew.-% diaminverlängerte Polyurethane elastifizierten Polymethacrylaten als Komponente von Reparaturmaterialien für Zahnprothesen, Brücken oder Kronen.

## Claims

1. Moulded dental fittings based on polymethacrylates, characterised in that polymethacrylates which have been elasticated by 0.5 to 12% by weight of non-crosslinked polyurethanes lengthened by diamines are used.

2. Process for the production of moulded dental fittings by the powder/liquid method, characterised in that fine-particled polymethyl methacrylates which have been elasticated by 0.5 to 12% by weight of non-crosslinked polyurethanes lengthed by diamines are used as the powder.

3. Process according to Claim 2, characterised in that elasticated fine-particled polymethacrylates which are obtained in the form of polymer beads by the bead polymerisation procedure are used as the powder.

4. Process according to Claim 2, characterised in that elasticated polymethyl methacrylates which are obtained as acrylate chips by a grinding process are used as the powder.

5. Process according to Claims 3 and 4, characterised in that non-elasticated methacrylic acid methyl ester polymers are mixed with the elasticated polymethyl methacrylates in order to establish the desired processability and processing range.

6. Use of polymethacrylates which have been elasticated by 0.5 to 12% by weight of polyurethanes lengthened by diamines as components of repair materials for dental prostheses, bridges or crowns.

## Revendications

1. Pièces moulées à usage dentaire à base de polyméthacrylates, caractérisées en ce qu'on utilise des polyméthacrylates qui sont élastifiés par 0,5 à 12% en poids de polyuréthannes non réticulés, prolongés par une diamine.

2. Procédé de production de pièces moulées à usage dentaire par le procédé poudre/liquide, caractérisé en ce qu'on utilise comme poudre des polyméthylméthacrylates finement divisés qui sont élastifiés par 0,5 à 12% en poids de polyuréthannes non réticulés prolongés par une diamine.

3. Procédé suivant la revendication 2, caractérisé en ce qu'on utilise comme poudre des polyméthacrylates élastifiés finement divisés qui sont obtenus sous forme de perles de polymérisat à la façon d'un procédé de polymérisation en perles.

4. Procédé suivant la revendication 2, caractérisé en ce qu'on utilise comme poudre des polyméthylméthacrylates élastifiés qui sont obtenus comme acrylates en morceaux par un procédé de broyage.

5. Procédé suivant les revendications 3 et 4, caractérisé en ce qu'on adjoint des polymérisats d'ester méthylique d'acide méthacrylique non élastifiés aux polyméthylméthacrylates élastifiés en vue de régler la transformabilité et la marge de transformation désirées.

6. Utilisation de polyméthacrylates élastifiés par 0,5 à 12% en poids de polyuréthannes allongés par une diamine comme composants de matériaux de restauration pour prothèses dentaires, bridges ou couronnes.